# EUROPÄISCHE PATENTANMELDUNG

(11) **EP 1 640 036 A1**
(43) Veröffentlichungstag der Anmeldung: **29.03.2006**
(21) Anmeldenummer: 04022731.6
(22) Anmeldetag: 23.09.2004
(51) Int. Cl.: A61N 2/00, A61B 5/05, G01V 9/00

(54) **Bioenergetisches Test- bzw. Diagnoseverfahren**

(71) Anmelder: Grundmeyer, Heinz, 38312 Börsshum (DE)
(72) Erfinder: Grundmeyer, Heinz, 38312 Börsshum (DE)
(74) Vertreter: Kirschner, Klaus Dieter

(57) **Zusammenfassung**

Außerhalb von Lebewesen erfolgendes bioenergetisches Test- bzw. Diagnoseverfahren zur Feststellung von Allergie-Empfindlichkeiten, dadurch gekennzeichnet, daß den Probanden ein Blutstropfen entnommen wird, welcher einer Spektralanalyse seines biologischen Frequenzspektrums unterworfen wird, daß die ermittelten einzelnen bioenergetischen Frequenzen digitalisiert und mit ihrer Intensität korrespondierenden einzelnen Organen zugeordneten Soll-Vergleichsfrequenzen einzeln in Beziehung gebracht werden, sowie die mit störungsbedingt verminderter Intensität mit ja oder nein kenntlich gemacht und dargestellt werden.

## Beschreibung

Die Erfindung betrifft ein außerhalb von Lebewesen eingesetztes bioenergetisches Test- bzw. Diagnoseverfahren auf Allergie-Erscheinungen und ein darauf abgestelltes Therapiemittel.

Bioenergetische Vorgänge spielen sich bioelektronisch in lebenden Zellen oder Zellverbänden in der Größenordnung von 0,002 V/m ab und wirken auf den einzelnen Organen spezifisch zuzuordnenden Frequenzen, welche die Zellen bzw. Zellverbände mit biologischen Informationen steuern. Im gesunden Zustand der Zellen befinden sich die Informationen bzw. Zellschwingungen in einem biologisch vorgegebenen Harmonie-Zustand.

Dieser Zustand kann durch unterschiedlichste körperliche Umstände oder Umwelteinfluß gestört werden.

Die erfindungsgemäße Allergie-Immun-Analyse basiert auf folgenden allgemein Bio-Physikalischen Grundlagen:
1. Die Vitalfunktionen unseres Körpers beruhen auf einer permanenten Kommunikation
   Gewebe und Organe oder Zellen und Zellverbände tauschen permanent Informationen aus. Diese Kommunikation geschieht durch äußerst geringe elektromagnetische Impulse.
2. Das Biophotonenfeld erzeugt Ordnungen
   Das Biophotonenfeld (morphogenetisches Feld, Eigenfeld, biologisches Feld) ordnet Moleküle im Protoplasma an. Das Biophotonenfeld ist elektromagnetischer Art, existiert und arbeitet über Zellgrenzen hinweg. Die elektromagnetischen Felder entstehen, wenn sich zwei elektrische Ladungen bewegen, senkrecht zu ihrer Bewegungsrichtung. (Fritz Adalbert Popp u.a.)
3. Die Informationen im Biophotonenfeld sind holografisch
   In einem Biophtotonenfeld sind alle Informationen gespeichert, die sich auch in den beteiligten Zellen befinden. Das holografische Informationssystem ist in jeder einzelnen Zelle vollständig enthalten. Daher können alle für eine (Alelrgie)-Analyse notwendigen Informationen auch in einem einzelnen Bruttropfen gefunden werden.
4. Die D N A reagiert auf menschliche Sprache
   Was die medizinische Hypnose bereits seit vielen Jahren praktiziert, aber man bislang in dieser Deutlichkeit nicht hat beweisen können, ist von zwei unterschiedlichen Forschern bestätigt worden: Sprache kann unsere Zellvorgänge beeinflussen.
   Pjotr Garjajev von der die Russischen Akademie der Wissenschaften fand heraus, daß die DNA nur zu 10 Prozent zum Aufbau von Genen dient. Die restlichen 90 Prozent werden zur Hyperkommunikation genutzt. Mit Hyperkommunikation ist gemeint, daß höhere Informationen ins menschliche Bewußtsein transportiert werden.
   Es wurde nämlich festgestellt, daß menschliche Sprachen in ihren Gesetzmäßigkeiten wie Syntax, Semantik und Grammatik dem genetischen Code folgen. Jede menschliche Sprache folgt diesem Grundmuster.
   Daß sich die genetischen Informationen durch Sprache beeinflussen ließ, konnten die russischen Forscher beweisen, als sie lebendige DNA-Substanz sprachmodulierten Laserlicht aussetzten.
   Zu einem ähnlichen Ergebnis kommt Nobelpreisträger Erich Kandel. Er fand heraus, daß Psychotherapie, also die Kommunikation zwischen Therapeut und Patient, Zellvorgänge nachhaltig beeinflussen kann. (Principles of Neural Science 1985): "Wenn wir miteinander sprechen, kommuniziert mein Gehirn mit ihrem, erzeugt dort anatomisch Veränderungen und umgekehrt".
5. Radiästhetische bzw. radionische Verfahren können die holistischen Informationen decodieren.
   Radiästethische bzw. radionische Verfahren können die Informationen in der Blutprobe decodieren. Radionik arbeitet im Bereich, in dem Materie und Bewußtsein aufeinander einwirken.
   Radionik arbeitet mit Skalarwellen. Skalarwellen haben die Eigenschaften, daß sie
   - Informationen übertragen
   - auf den Atomkern wirken
   - Materie durchdringen
6. Wenn Sprache aber DNA beeinflussen kann, muß im Rückschluß die DNA-Information auch in Sprache wieder umgesetzt werden können Das ist der Fall, indem mit den "richtigen Fragen" (Ja/Nein-Fragen) die Informationen abgefragt werden.
7. Derartige Frequenzen bzw. bioenergetische Frequenzen sind heutzutage auch mit klassischen spektraanalytischen Verfahren meßbar, also auch die Frequenz zu gestörter Zellen bzw. Zellverbände mittels eines Frequenzanalysators.
   Mit einem physikalischen Frequenzanalysator lassen sich Schwingungen beliebiger Frequenzen und Träger wie Wasser und Alkohole dauerhaft aufprägen.
8. Zwischen einem entnommenen Blutstropfen und dem zugehörigen Körper bleibt die kommunikative Verbindung bestehen. Diese bleibt mit dem Körper über Raum und Zeit bestehen, d. h. jedwede im Körper erfolgte Veränderung kann auch in Blutstropfen oder Speichel physikalisch nachgewiesen werden.

Das diese Verbindung besteht, beweisen zwei physikalische Versuche:
A: Quanten-Teleportation: Informationsaustausch ist jenseits herkömmlicher Übertragung möglich.
   Am 18. Dezember 2003 gelang im Labor des Forschungszentrums TIM-Lab der Donau-Universität Krems ein öffentliches Experiment zu einem völlig neuen Verfahren der drahtlosen Datenübertragung.
   Dr. Hartmut Müller, Leiter des Instituts für Raum-Energie-Forschung in Wolfratshausen (Deutschland), demonstrierte, daß eine Datenübertragung zwischen zwei Notebooks in Deutschland und Krems ohne herkömmliche Übertragungsverfahren (wie Internet, Telefon, WLAN, etc.) und ohne Verwendung zusätzlicher Geräte rein softwarebasierend möglich ist.
   Das von Dr. Müller in zwanzigjähriger Forschung entwickelte Verfahren "Global-Scaling Quantum Teleportation" (GSQT) ermöglicht diese Datenübertragung über das kosmische Hintergrundrauschen des in aller Materie enthaltenen Vakuums. Entgegen den Vorstellungen der klassischen Physik haben die Eigenschwingungen des Vakuums kein chaotisches, sondern ein harmonisches Spektrum. Deshalb kann man diese Schwingungen im Vakuum-Resonanzverfahren modulieren und für Quantenteleportation nutzen. (http://www.donau-uni.ac.at/)
B. Zwillingsphotonen beweisen: Für immer miteinander verbunden
   Ein anderes Experiment nutzte Zwillingsphotonen zur Übertragung von verschlüsselten Daten. Die Übertragung eines Bildes von einem zum anderen Computer gelang an der Universität Wien.

Ursache für Allergien sind Fehlinformationen und treten zunehmend auf, wobei allein in Deutschland jeder Dritte unter Allergien leidet.
die derzeit verbreiteten Test-Verfahren sind in ihrer Aussagefähigkeiten über den Ursprung der jeweiligen Allergien nur begrenzt und aufwendig und bedingen oft auch eine umständliche Belastung bzw. Handhabung für den Allergiker.

Dazu sind beispielsweise als Standard-Untersuchung der Prick-Test zu rechnen, der trotz einer Vielzahl von Nadelstichen nur eine vor allem lokalbegrenzte Aussagekraft hat sowie der Intrakutan-Test, der selten in einer normalen Arztpraxis durchführbar ist.

Der Radio-Allergie-Sorbent-Test (RAST) basiert auf der Bestimmung von Antikörpern (Reagine) gegen bestimmte allergieauslösende Substanzen.

Beim konjunktivalen Provokationstest wird ein vermitteltes Allergen auf die Haut und beim Epikutantest werden Pflaster mit unterschiedlichen einzelnen vermuteten Allergenen auf den Rücken geklebt, während man beim Yorktest ausschließlich proteinspezifische IgG-Reaktionen ermitteln kann und keine klassischen IgE-Allergien.

Das Besondere am erfindungsgemäßen Allergie-Immun-Test und einem darauf basierenden Therapiemittel ist seine universelle Anwendbarkeit sowohl auf die volle Spektrumsbreite aller denkbaren Allergien und allergie-auslösende Substanzen als auch die einfache sowie praktische Weise der Verfahren durchführen. Auch kann statt Blut auch ebenso Speichel analysiert werden.

### Spezielle Hintergründe des Verfahrens

Dem erfinderischen Verfahren liegen folgende Grundlagen zugrunde:
1. Allergien sind Fehlinformationen, die in Zellen des Körpers gespeichert werden.
2. Diese Zellinformationen können sichtbar gemacht werden.
3. Die unterschiedlichen Ausbildungen (Phänotypen) von Allergien sind auf drei Grundallergien zurück zu führen. Die Grundallergien sind Unverträglichkeiten von Weizen, Gliadin und Kuhmilcheiweiß.
4. Alle anderen Allergien satteln auf den Grundallergien auf bzw. sind Ausdruck verschiedener energetischer Fehlinformationen, die sich in Folge der unbehandelten Grundallergien verlagert und verstärkt haben.
5. Eine erfolgreiche Allergiebehandlung erfordert in der Regel eine stufenweise Beseitigung von Fehlinformationen.
6. Bei der Beseitigung von Allergien kann es zu Verlagerungen und energetischen Blockaden an anderen Stellen kommen. Der Grund dafür: Das Gesamtsystem versucht sich selbst zu erhalten, indem es versucht, die Fehlinformationen zu korrigieren. Da wir nur ein gewisses Maß an Energie zur Verfügung haben, kann er eben nur einen Teil dessen für diese Aufgabe verwenden. Den Rest braucht er, um das System für die täglichen Dinge am Laufen zu erhalten. Den für den Körper weniger wichtigen Fehlinformationen widmet er sich erst, wenn die vorhandenen Haupt-Fehlinformationen beseitigt sind. Diese weiteren Fehlinformationen werden dann in einem weiteren Analysetest nachgewiesen.

Allergien entstehen aus Fehlinterpretationen im Kommunikationsprozess on Zellen und Zellverbänden. Das heißt: Bestimmte Zellverbände halten ein mit der Nahrung aufgenommenes Eiweiß für eine Bedrohung und reagieren mit Abwehr.

Wichtiger noch als der energetische Austausch zwischen den Zellen und Organen ist der Informationsaustausch. Dieser geschieht durch Veränderung der elektrischen Spannungen der Zellmembran. Die Spannung ist Folge der Veränderung ihrer Durchlässigkeit für Ionen, die sich auf der Innen- und Außenseite der Membran befinden.

Damit Ionen, zum Beispiel Kalium- oder Natrium-Ionen, vom Zellinnern in die Umgebung können, nutzen sie winzige Ionen-Kanäle.

Die Nobelpreisträger Sakmann und Neher haben entdeckt, daß bereits minimale Feldstärken von 0,002 V/m zur Steuerung dieser Prozesse im Körper ausreichen. Außerdem konnten sie nachweisen, daß viele Krankheiten ganz oder teilweise auf einer defekten Regulierung des lonenflusses basieren.

Sehr viele Körperfunktionen, vor allem Sensorik, Motorik, Sekretion werden durch die lonenkanal-Kommunikation gesteuert.

Jede einzelne Zelle wird durch Minus- und Plus-Polarität am Leben erhalten. Der Plus-Pol befindet sich im Zellkern und der Minus-Pol in der Zellhülle. Dabei stehen die positive Spannung des Zellkerns und die negative Spannung der Hülle bei der gesunden Zelle in einem ausgeglichenen Verhältnis zueinander. Durch diese Spannung entsteht elektrische Energie, die äußerst minimal ist, aber ausreicht, um Prozesse in Lebewesen zu steuern.

Erzeugt wird diese Energie in Mitochondrien. Mitochondrien sind die "Kraftwerke" in unseren ca. 100 Milliarden Körperzellen zur Erzeugung von Bioelektrizität. Gespeist werden die Mitochondrien durch die Sonne bzw. durch deren Lichtteilchen (Photonen). Ohne Sonne, ohne Photonen ist daher ein Leben dauerhaft nicht möglich.

Zellen sind in Verbänden oder Gruppen gegliedert. Dabei ist das Aufgabengebiet für jede Zellgruppe genau abgesteckt. Sie untersteht einem übergeordneten Befehlsgeber, der über Steuerungsimpulse vorprogrammierte Arbeiten und Aktionen aktiviert oder hemmt vorbestimmte Arbeiten und Arbeiten und Aktionen aktiiviert oder hemmt und bremst. Die Zellgruppen sind also ständig in Bereitschaft und tauschen u. a. über die bioelektrischen Ströme Informationen aus.

Für derartige Messungen wurden bisher ein Biotensor, Rute oder Pendel erfolgreich eingesetzt.

Nunmehr sind die Meßwerte der zwar schwachen aber als physikalische hochfrequente elektromagnetische Schwingungsstrahlung mit klassischen physikalischen Meßgeräten wie z. B. Bio-Photonen- oder spektrographische Geräte, und zwar zur Erhöhung der Treffsicherheit gleich als ja/nein-Differenzierung bzw. Digitalisierung.

Ein radionisches Analysegerät, das mit einem handelsüblichen Computer gekoppelt wird und mit Hilfe des oben beschriebenen Analyseplans die einzelnen potentiellen Fehlinformationen bzw. Blockaden lokalisiert, ist für die Analyse auch geeignet und vereinfacht die Analyse.

In der Analyse ist dabei ein Computer der Empfänger. Er nimmt Signale des Frequenzmeßgeräts oder vom Radionik-Gerät auf, die von der zu analysierenden Probe ausgehen.
Wenn mit oben genannten Verfahren ein Bluttropfen analysiert wird, so geht man von folgenden Voraussetzungen aus:
1. Im Bluttropfen sind alle das Lebewesen betreffenden, relevanten Informationen vorhanden
2. Die relevanten Informationen können analysiert werden. Mit dem radionischen Verfahren läßt sich auch das morphogenetische Feld, das ein Objekt umgibt, analysieren.

Beim Radionik-Gerät übernimmt das Gerät im Zusammenhang mit dem Analyseplan die Funktion einer instrumentellen Biokommunikation.

Anhand des Analyseplans wird abgefragt, ob beispielsweise im 1. Brustwirbel, im 2. Brustwirbel, im 3. Brustwirbel usw. eine Blockade vorliegt. Dabei geben eindeutige Pendel- oder Rutenbewegung Ja- oder Nein-Antworten.

Mit eindeutigen Ja/Nein-Fragen wird der Analyseplan 2 abgearbeitet bis die für die Allergiebehandlung wichtigen Meridiane, Drüsen und der Wirbelsäulenapparat auf Fehlfunktionen und Energieblockaden untersucht worden sind.

### Analyse

1. Vor der Analyse mit dem Frequenzmeß-Gerät müssen evtl. noch vorhandene energetische Informationen gelöscht werden.
2. Auf die linke Schale des Gerätes wird nun die Blutprobe gelegt.
3. Das Analyseprogramm wird jetzt gestartet.
4. Die entsprechenden Fehlerquellen (z. B. Drüsenfehlfunktion, Epiphyse oder Toxische Belastungen, Kupfer) werden im Analysebogen mit Kreuzchen markiert.

In einem Hochfrequenzgenerator werden dann die als gestörte Frequenzen bzw. Informationen auf ein Trägermedium aufgeprägt, mit dessen Zuführung in den zu behandelnden lebendigen Körper als Therapiemittel entweder ein oder in bestimmten Zeitabständen mehrmals in oraler oder subkutaner .Applikation dem zu behandelnden Körper zugeführt wird. Damit werden die gestörten Zellinformationen entstört bzw. neu aufgebaut.

Für das erfindungsgemäße Diagnoseverfahren wird dem Patienten eine Blut- oder Speichelprobe entnommen, wobei auch ein sehr kleiner Blutstropfen ausreicht.

Dieser wird in einem Frequenz-Meßgerät einer Frequenz-Analyse über den gesamten bioelektronischen Frequenzbereich unterworfen und die jeweils gemessenen Frequenzwerte für die einzelnen Organe festgestellt, in .bevorzugt digitalisierter Weise gemessen und auf die Meßalternative ja/nein den für die Frequenzwerte zuzuordnenden Organe verglichen werden, wie z. B. den Drüsenfunktionen.

### Analyseplan 2: Potentielle bioenergetische Blockaden und Drüsenfehlfunktionen

**Drüsenfehlfunktionen**

| **7 Inkretorische Drüsen** | **7 Nebendrüsen** |
|---|---|
| Epiphyse | Thalamus |
| Himanhangdrüse | Hypothalamus |
| Schilddrüsen | Nebenschilddrüsen |
| Thymusdrüse und Milz | Kerne hinter der Thymusdrüse |
| Nieren | Neben-Nieren |
| Bauchspeicheldrüse | Alpha- & Beta-Rezeptoren Pankrea |
| | kern |
| Geschlechtsdrüsen | Keimdrüsen |

Ebenso werden die gemessenen Frequenzwerte bzw. ja/nein mit den Kenndaten des folgenden Blokkadeplans verglichen:

**Bioenergetische Blockaden**

| **Wirbelsäulen-Blockaden** | **Blockaden in den Meridianen** | | |
|---|---|---|---|
| Halswirbel | Blasen-Meridian | Dickdarm Meridian | |
| Atlas | Nieren-Meridian | Lungen-Meridian | |
| C1 | Gallenblasen-Meridian | Dreifacher Erwärmer | |
| C2 | Leber-Meridian | Herzkreislauf-Meridian | |
| C3 | Magen-Meridian | Dünndarm-Meridian | |
| C4 | Milz-Meridian | Herz-Meridian | |
| C5 | **Blockaden Ausscheidungsorgane** | | |
| | **Nieren** | **Haut** | |
| C6 | Darm | Lunge | |
| C7 | Leber | Schleimhäute | |
| Brustwirbel | chem. Medikamentöse Belastungen | | |
| | Antibiotika | Corticoide | |
| TH1 | **Penicilin** | **Impfstoffe** | |
| TH2 | **Antiseptika** | **Anaestika** | |
| TH3 | Phychopharmaka | Globuli | |
| TH4 | Antimykotika | Nosoden | |
| TH5 | **Toxische Belastungen** | | |
| TH6 | Aluminium | Kobalt | Tanal |
| TH7 | Amalgam | Kupfer | Thalium |
| TH8 | Arsen | Lithium | Titan |
| TH9 | Berylium | Magenesium | Vanadium |
| TH10 | Blei | Mangan | Wolfram |
| TH11 | | | |
| TH12 | | | |
| **Lendenwirbel** | Chrom | Molybdän | Zink |
| L2 | Eisen | Nickel | Zinn |
| L3 | Galium | Osmium | |
| L4 | Gold | Palladium | |
| L5 | Indium | Quecksilber | |
| Steißbein | Iridium | Rubidium | |
| Kreuzbein | Cadmium | Silber | |
| Beckenschiefstand | | | |
| | **Psychosomatische Blockaden** | | |
| | im Parasympathikus | negative Engramme | |
| | (*7)(*2)(3*) | (*2)(4*) | |
| | Psychische (*5) | Posttraumatische (*6) | |

| | | | |
|---|---|---|---|
| Erklärungen: (*1) 7 inkretorische Drüsen sind die Arbeitsorgane und werden gesteuert von 7 Nebendrüsen | | | |
| (*2) die im zentralen Nervensystem hinterlassene Gedächtnisspur (Muster) (mnemische Spur) eines Reiz- oder Erlebniseindrucks, die dessen geistige Reproduktion zu einem späteren Zeitpunkt ermöglicht. | | | |
| (*3) aus unbegründeten Ängsten produziert. Können Phobien, Höhenängste etc. auslösen. | | | |
| (*4) meist aus der Jugend, wenn als Kind z. B. "ungerecht" behandelt wurde. | | | |
| (*5) alte Muster von Vorfahren, die Erlebnisse nicht aufgelöst haben. | | | |
| (*6) oft ein wiederkehrender, nicht aufgelöster Traum | | | |
| (*7) Parasympathikus = Unterbewußtsein | | | |

Aufgabe der Erfindung ist ein handhabungsmäßig außerordentlich vereinfachter Test- bzw. Diagnoseverfahren außerhalb von Lebewesen und die Herstellung eines darauf abgestellten Therapiemittels insbesondere für Allergieerkrankungen auf frequenzmedizinischer Grundlage.

Dafür wird dem Probanden lediglich ein Blutstropfen oder auch eine Speichelprobe zur Feststellung eines digitalisierten Frequenzspektrums davon entnommen und gemäß der aufgrund des Allergiebefalls ermittelten Frequenzstörungen ein darauf abgestelltes, durch Frequenzaufprägung auf ein Trägermedium hergestellt, dessen aufgeprägte Frequenzen die Minderung der Schwächung der Allergie geschwächten Biofrequenzen wieder behebt.

## Patentansprüche

1. Außerhalb von Lebewesen erfolgendes bioenergetisches Test- bzw. Diagnoseverfahren zur Feststellung von Allergie-Empfindlichkeiten, **dadurch gekennzeichnet, daß** den Probanden ein Blutstropfen entnommen wird, welcher einer Spektralanalyse seines biologischen Frequenzspektrums unterworfen wird, daß die ermittelten einzelnen bioenergetischen Frequenzen digitalisiert und mit ihrer Intensität korrespondierenden einzelnen Organen zugeordneten Soll-Vergleichsfrequenzen einzeln in Beziehung gebracht werden, sowie die mit störungsbedingt verminderter Intensität mit ja oder nein kenntlich gemacht und dargestellt werden.

2. Test- bzw. Diagnoseverfahren, **dadurch gekennzeichnet, daß** die gegenüber den Vergleichsfrequenzen als energetisch gemindert festgestellten Frequenzen dem Probanden in Form von zusätzlich verstärkten Frequenzen mittels eines Trägers, wie beispielsweise Wasser oder einer alkoholhaltigen Flüssigkeit, wieder zugegeben werden.

3. Therapiemittel gegen Allergieempfindlichkeiten. bei dem die insbesondere nach dem Verfahren nach Anspruch 1 oder 2 ermittelten intensitäts- geschwächten bioenergetischen Frequenzen einem Träger wie Wasser oder alkoholhaltiger Flüssigkeit in gesteigerter Intensität aufgeprägt sind.
